# EUROPEAN PATENT APPLICATION

(11) **EP 4 353 210 A1**
(43) Date of publication of application: **17.04.2024**
(21) Application number: 21951956.8
(22) Date of filing: 13.12.2021
(51) Int. Cl.: A61F 13/49, A61F 13/496, A61F 13/64

(54) **PANTS-TYPE ABSORBENT ARTICLE**

(30) Priority: 30.07.2021 CN 202110871047
(71) Applicant: Unicharm Corporation, Ehime 799-0111 (JP)
(72) Inventor: FUJIMOTO, Kazuya, Kanonji-shi, Kagawa 769-1602 (JP); UDAKA, Hikari, Kanonji-shi, Kagawa 769-1602 (JP); ITO, Yoshihiro, Kanonji-shi, Kagawa 769-1602 (JP)
(74) Representative: Staeger & Sperling Partnerschaftsgesellschaft mbB
(86) International application number: PCT/JP2021/045859
(87) International publication number: WO 2023/007763

(57) **Abstract**

A pants-type absorbent article (1) comprising an absorbent body (10) and a belt portion (30) connecting between a front-side upper end (10eLf) and a back-side upper end (10eLb) of the absorbent body (10), wherein the belt portion (30) is joined with the absorbent body (10) via a joint part (40) that slopes from an inner side toward an outer side in the right-left direction and from a top side toward a bottom side in the vertical direction starting at a beltline opening (BH) toward leg-side openings (LH), and, when said pants-type absorbent article (1) in an extended state is viewed in the front-back direction, the joint part (40) includes a portion (40fp) that projects inward in the right-left direction as compared with a straight line connecting an upper end (40fea) and a lower end (40feb) in the vertical direction of the joint part (40).

## Description

### FIELD

The present invention relates to an underpants-shaped absorbent article.

### BACKGROUND

An underpants-shaped absorbent article has been conventionally known in which an absorbent main body and a pair of belt portions are joined by diagonally-inclined joining portions. For example, Patent Document 1 discloses a diaper 10 (underpants-shaped disposable diaper) having a body 20 (absorbent main body) and a stretchable panel 30 (belt portion). In this diaper 10, the panel 30 is joined onto the front edge 15 of the body 20 by a pair of diagonally-inclined front attachment lines 21, and the panel 30 is joined onto on the back edge 17 of the body 20 by a pair of diagonally-inclined back attachment lines 22. Thus, a waist opening 16 and leg opening edges 12 are formed.

### [CITATION LIST]

### [PATENT LITERATURE]

[Patent Document 1] Japanese Patent Application Publication No. H10-179639

### SUMMARY

### [TECHNICAL PROBLEM]

In an underpants-shaped diaper according to Patent Document 1, at the time of putting it on, the belt portion is pulled diagonally upward, thereby pulling the absorbent main body toward the wearer's crotch portion. At that time, a force concentrates on the lower ends of the diagonally-inclined joining portions that join the absorbent main body and the belt portion, and this makes the joining portion likely to break from the lower end portions, causing a risk of separating the belt portion from the absorbent main body.

The present invention was achieved in light of conventional problems such as that described above, an aspect of the present invention is to provide an underpants-shaped absorbent article in which an absorbent main body and a belt portion are joined by diagonally-inclined joining portions and in which breakage of the joining portions is suppressed when putting on.

### [SOLUTION TO PROBLEM]

A main aspect of the present invention of achieving the above-described aspect is an underpants-shaped absorbent article having a vertical direction, a lateral direction, and a front-back direction that intersect with each other, the underpants-shaped absorbent article including: an absorbent main body; and a belt portion that connects a front upper end portion and a back upper end portion of the absorbent main body, the underpants-shaped absorbent article having a waist opening and a pair of leg openings, the belt portion being joined to the absorbent main body by a joining portion that inclines from the waist opening towards the leg opening from above to below in the vertical direction and from inside to outside in the lateral direction, when the underpants-shaped absorbent article in a stretched state is viewed along the front-back direction, the joining portion having a portion that protrudes inward in the lateral direction with respect to a straight line that connects a vertical upper end and a vertical lower end of the joining portion.

Features of the present invention other than the above will become clear by reading the description of the present specification with reference to the accompanying drawings.

### [ADVANTAGEOUS EFFECT OF THE INVENTION]

According to the present invention, in an underpants-shaped absorbent article in which an absorbent main body and a belt portion are joined by diagonally-inclined joining portions, it is possible to suppress breakage of the joining portions when putting on.

### BRIEF DESCRIPTION OF DRAWINGS

FIG. 1 is a schematic front view of a diaper 1 as seen from the front side, and a schematic cross-sectional view of a waist opening BH as seen from above.
FIG. 2 is a plan view of the diaper 1 in a state where an absorbent main body 10 and a belt portion 30 are stretched in the longitudinal direction.
FIG. 3A is a schematic cross-sectional view of the diaper 1 in the state shown in FIG. 2, taken along a line A-A.
FIG. 3B is a schematic cross-sectional view of the diaper 1 in the state shown in FIG. 2, taken along a line B-B.
FIG. 4 is a diagram illustrating the relationship of forces acting on joining portions 40 when putting on a conventional underpants-shaped diaper 5 as a comparative example.
FIG. 5 is a diagram illustrating the relationship of forces acting on the joining portions 40 when putting on the underpants-shaped diaper 1 of the first embodiment.
FIG. 6 is a perspective view showing a leg opening LH of the diaper 1 during a putting-on operation.
FIG. 7 is a diagram illustrating the arrangement of convex portion 40fp provided in the joining portion 40.
FIG. 8 is an enlarged plan view illustrating the structure of the joining portion 40.
FIG. 9 is a plan view illustrating a case where the discontinuous portions are provided in the joining portion 40.
FIGS. 10A and 10B are diagrams showing modified examples of the arrangement of sealing portions 41 that constitute the joining portion 40.
FIG. 11 is a schematic plan view illustrating a modified example of the joining portions 40.
FIG. 12 is a schematic plan view illustrating another modified example of the joining portions 40.
FIG. 13 is a schematic front view of a diaper 2 of the second embodiment as seen from the front side.
FIGS. 14A and 14B are diagrams illustrating an example of a method for forming a projected portion 10t and a projected portion 30t.
FIG. 15 is a perspective view showing a leg opening LH of the diaper 2 during the putting-on operation.

### DESCRIPTION OF EMBODIMENTS

At least the following matters will be clear with the description of this specification and the attached drawings.

An underpants-shaped absorbent article having a vertical direction, a lateral direction, and a front-back direction that intersect with each other, the underpants-shaped absorbent article including: an absorbent main body; and a belt portion that connects a front upper end portion and a back upper end portion of the absorbent main body, the underpants-shaped absorbent article having a waist opening and a pair of leg openings, the belt portion being joined to the absorbent main body by a joining portion that inclines from the waist opening towards the leg opening from above to below in the vertical direction and from inside to outside in the lateral direction, when the underpants-shaped absorbent article in a stretched state is viewed along the front-back direction, the joining portion having a portion that protrudes inward in the lateral direction with respect to a straight line that connects a vertical upper end and a vertical lower end of the joining portion.

According to the underpants-shaped absorbent article, when pulling up the belt portions at the time of putting it on, a portion of the joining portion that is located below the lateral-inwardly-protruding portion (convex portion) rises up from the wearer's skin side to the non-skin side, and this makes the lower end of the joining portion not come into contact with the wearer's skin. Therefore, the force for pulling up the belt portion is less likely to act on the lower end of the joining portion, making the starting point of a tear less likely to be formed at the lower end. This makes the joining portion less likely to tear, making it possible to prevent the belt portion and the absorbent main body from separating.

In such an underpants-shaped absorbent article, it is desirable that the joining portion has the portion that protrudes inward in the lateral direction, the portion being located in the vertical direction below an intermediate position between an upper end of the joining portion and a lower end of the joining portion.

According to the underpants-shaped absorbent article, compared to the case where the convex portion of the joining portion is provided above the intermediate position in the vertical direction, the area of a portion where the belt portion rises up from the wearer's skin when putting on the absorbent article decreases. Therefore, it is possible to suppress the deterioration of the fit. Further, since the convex portion is arranged below the intermediate position in the vertical direction, it is less likely to suppress a contractive force in the vicinity of the upper end of the belt portion, and it is possible to prevent the belt portion from slipping down.

In such an underpants-shaped absorbent article, it is desirable that, of the joining portion, at least a part of the portion that protrudes inward in the lateral direction is curved.

According to the underpants-shaped absorbent article, corners are less likely to be formed in the curved portion of the joining portion, and this suppresses the force for pulling up the belt portion during the putting-on operation from concentrating and acting. In addition, in the portion where the joining portion is curved, the length of the joining portion itself becomes longer compared to the case where the joining portion is formed in a straight line, and therefore the force is likely to be distributed. This can prevent the joining portion from tearing.

In such an underpants-shaped absorbent article, it is desirable that the joining portion includes a plurality of sealing portions that are arranged in a discrete manner, and that at least one of the plurality of sealing portions has a curved outline, below a center position of the at least one of the plurality of sealing portions in the vertical direction.

According to the underpants-shaped absorbent article, the plurality of small sealing portions are arranged at intervals, and therefore the force acting on the joining portion is likely to be distributed, and this can make the joining portion less likely to tear. In addition, the outline of the lower part of the sealing portion is curved and no corners are formed therein, the diagonally-upward force acting on the joining portion is less likely to concentrate in one location, making the sealing portion less likely to peel off. This can prevent the joining portion from tearing.

In such an underpants-shaped absorbent article, it is desirable that the joining portion has a discontinuous portion between the vertical upper end and the vertical lower end.

According to the underpants-shaped absorbent article, even if tearing occurs in a certain part of the joining portion, the effect of the tearing is stopped at the discontinuous portion, and therefore it prevents the tearing from spreading throughout the joining portion. This prevents the entirety of the joining portion from tearing.

In such an underpants-shaped absorbent article, it is desirable that the joining portion has a portion where a width in a region located vertically below the portion that protrudes inward in the lateral direction is greater than a width in a region located vertically above the portion that protrudes inward in the lateral direction.

According to the underpants-shaped absorbent article, when pulling up the belt portion at the time of putting on, the force that acts on the joining portion from below to above in the vertical direction can be received by the portion in the lower portion of the joining portion. Therefore, the force that acts on the lower portion of the joining portion is likely to be distributed in the width direction, and it can make it likely to prevent the joining portion from tearing.

In such an underpants-shaped absorbent article, it is desirable that the joining portion includes a plurality of sealing portions that are arranged in a discrete manner, and that, concerning a density per unit area of the sealing portion, the joining portion has a portion where the density in a region located vertically below the portion that protrudes inward in the lateral direction is greater than he density in a region located vertically above the portion that protrudes inward in the lateral direction.

According to the underpants-shaped absorbent article, when pulling up the belt portion at the time of putting on, the force that acts on the joining portion from below to above in the vertical direction can be received by a lower portion of the joining portion where the welding strength is high because the density of sealing is high. Therefore, it is possible to resist the force acting on the lower portion of the joining portion, enabling to make the joining portion less likely to tear.

In such an underpants-shaped absorbent article, it is desirable that the joining portion has a joined portion and an unjoined portion, in an end located on a non-skin side.

According to the underpants-shaped absorbent article, the stiffness of the joining portion in the non-skin-side end is not excessively high, and this makes discomfort less likely to occur if the wearer touches the joining portion. Further, since the absorbent main body and the belt portion are joined in at least a part of the non-skin-side end of the joining portion, this makes it likely to prevent the separating of them.

In such an underpants-shaped absorbent article, it is desirable that, in at least a part of an upper end portion of the absorbent main body, the underpants-shaped absorbent article has an absorbent-main-body sealing portion that joins only a sheet member that constitutes the absorbent main body.

According to the underpants-shaped absorbent article, by the presence of the absorbent-main-body sealing portion in the upper end portion of the absorbent main body, it is possible to suppress fraying between sheet members (e.g., the top sheet and the back sheet) that constitute the absorbent main body, and to prevent the opening of the absorbent main body in the upper end portion.

In such an underpants-shaped absorbent article, it is desirable that a high basis-weight region is provided in at least a part of a lower end portion of the belt portion, the high basis-weight region being a region where a basis weight is higher than in a remaining portion.

According to the underpants-shaped absorbent article, when joining the belt portion and the absorbent main body by the joining portion, the number of joining material in the high basis-weight region is larger that in a remaining regions, and this can make it likely to increase the welding strength. Therefore, when pulling up the belt portion during the putting-on operation, the joining portion is less likely to tear in the high basis-weight region of the belt portion, and this makes it likely to suppress the separation of the belt portion and the absorbent main body.

In such an underpants-shaped absorbent article, it is desirable that a multi-stacked region is provided in at least a part of a lower end portion of the belt portion, the multi-stacked region being a region where a number of sheet members that are stacked and constitute the belt portion is larger than in a remaining portion.

According to the underpants-shaped absorbent article, when joining the belt portion and the absorbent main body by the joining portion, the number of joining material in the multi-stacked region is larger that in a remaining regions, and this can make it likely to increase the welding strength. Therefore, when pulling up the belt portion during the putting-on operation, the joining portion is less likely to tear in the multi-stacked region of the belt portion, and this makes it likely to suppress the separation of the belt portion and the absorbent main body.

In such an underpants-shaped absorbent article, it is desirable that, in the multi-stacked region, the joining portion has a portion that protrudes inward in the lateral direction.

According to the underpants-shaped absorbent article, in the belt portion, by arranging the convex portion in the multi-stacked region, in which the number of stacked sheets is larger and the stiffness is high compared to the remaining regions, it is possible to make the joining portion less likely to tear even in the case where a large force acts on the convex portion.

In such an underpants-shaped absorbent article, it is desirable that the belt portion has a stretchable sheet member and a linear elastic member both of which stretch and contract in the lateral direction, and that the joining portion has a portion that protrudes inward in the lateral direction, vertically below a region of the belt portion where the stretchable sheet member and the linear elastic member are stacked.

According to the underpants-shaped absorbent article, the portion of the belt portion where the stretchable sheet member and the linear elastic member are stacked is a region where the stress is strongest when pulling up the belt portion during the putting-on operation. Therefore, providing the convex portion below this portion makes an excessively large force less likely to act on the convex portion, making it possible to make the belt portion less likely to tear.

In such an underpants-shaped absorbent article, it is desirable that, in a region located in the vertical direction below the lower end of the joining portion, the underpants-shaped absorbent article has a portion where the belt portion and the absorbent main body are continuous.

According to the underpants-shaped absorbent article, between the lower end of the joining portion and the leg opening, there is a part of the base material (nonwoven fabric, etc.) that constitutes the belt portion and the absorbent main body, and therefore the force when pulling up the belt portion at the time of putting on is less likely to act on the lower end of the joining portion. This suppresses the formation of a tear starting point in the lower end of the joining portion, and can make the joining portion further less likely to tear.

An underpants-shaped absorbent article having a vertical direction, a lateral direction, and a front-back direction that intersect with each other, the underpants-shaped absorbent article including: an absorbent main body; and a belt portion that connects a front upper end portion and a back upper end portion of the absorbent main body, the underpants-shaped absorbent article having a waist opening and a pair of leg openings, the belt portion being joined to the absorbent main body by a joining portion that inclines from the waist opening towards the leg opening from above to below in the vertical direction and from inside to outside in the lateral direction, when the underpants-shaped absorbent article in a stretched state is viewed along the front-back direction, in a vertical lower end portion of the joining portion, the underpants-shaped absorbent article has a portion where at least one of the absorbent main body and the belt portion protrudes toward a side of the leg opening.

According to the underpants-shaped absorbent article, when pulling up the belt portions at the time of putting it on, the projected portion that is formed in at least one of the absorbent main body and the belt portion rises up from the wearer's skin side to the non-skin side, and this makes the lower end of the joining portion not come into contact with the wearer's skin. Therefore, the force for pulling up the belt portion is less likely to act on the lower end of the joining portion, making the starting point of a tear less likely to be formed at the lower end. This makes the joining portion less likely to tear, making it possible to prevent the belt portion and the absorbent main body from separating.

An underpants-shaped absorbent article having a vertical direction, a lateral direction, and a front-back direction that intersect with each other, the underpants-shaped absorbent article including: an absorbent main body; and a belt portion that connects a front upper end portion and a back upper end portion of the absorbent main body, the underpants-shaped absorbent article having a waist opening and a pair of leg openings, the belt portion being joined to the absorbent main body by a joining portion that inclines from the waist opening towards the leg opening from above to below in the vertical direction and from inside to outside in the lateral direction, the joining portion being arranged so that during putting on a gap is formed between a lower end portion of the joining portion and a wearer's skin by the lower end portion of the joining portion rising up from a wearer's skin side to a wearer's non-skin side.

According to the underpants-shaped absorbent article, when pulling up the belt portions at the time of putting it on, a portion of the joining portion that is located below the lateral-inwardly-protruding portion (convex portion) rises up from the wearer's skin side to the non-skin side, and this makes the lower end of the joining portion not come into contact with the wearer's skin. Therefore, the force for pulling up the belt portion is less likely to act on the lower end of the joining portion, making the starting point of a tear less likely to be formed at the lower end. This makes the joining portion less likely to tear, making it possible to prevent the belt portion and the absorbent main body from separating.

### First embodiment

### Basic configuration of diaper 1

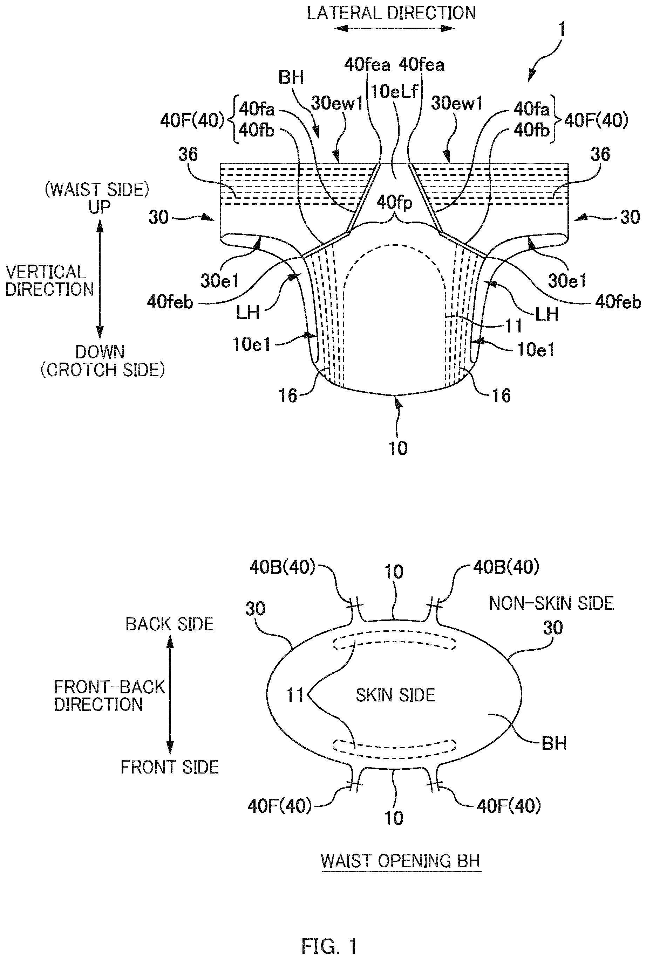

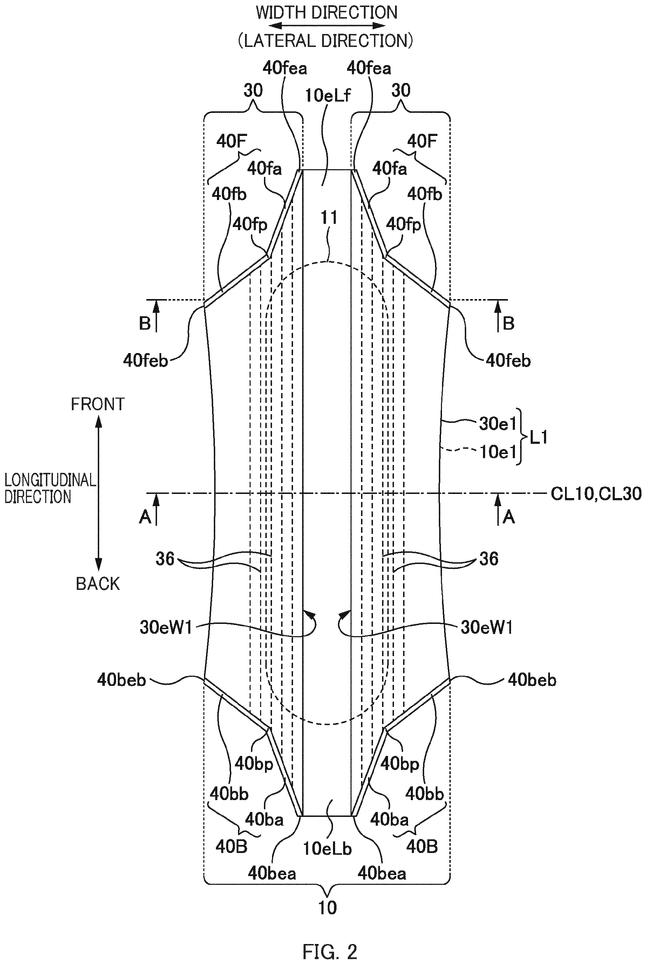

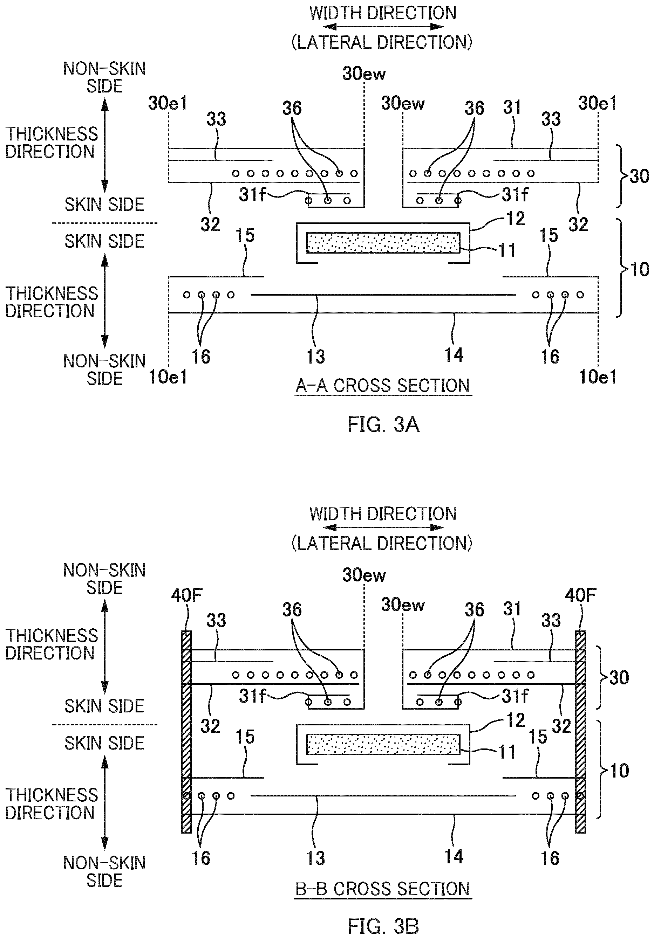

The following describes the basic configuration of an underpants-shaped disposable diaper 1 (hereinafter also referred to as "diaper 1") by way of example of an underpants-shaped absorbent article according to the first embodiment. FIG. 1 is a schematic front view of the diaper 1 as seen from the front side, and a schematic cross-sectional view of a waist opening BH as seen from above. FIG. 2 is a plan view of the diaper 1 in a state where an absorbent main body 10 and a belt portion 30 are stretched in the longitudinal direction. FIG. 3A is a schematic cross-sectional view of the diaper 1 in the state shown in FIG. 2, taken along a line A-A. FIG. 3B is a schematic cross-sectional view of the diaper 1 in the state shown in FIG. 2, taken along a line B-B.

The diaper 1 has a "vertical direction," a "lateral direction," and a "front-back direction," which are orthogonal to each other in an underpants-shaped state shown in FIG. 1. When wearing the diaper 1, the upper side in the vertical direction is the "waist side" of the wearer, and the lower side in the vertical direction is the "crotch side" of the wearer. Also, the front side in the front-back direction is the "stomach side" of the wearer, and the back side in the front-back direction is the "back side" of the wearer.

Furthermore, at the final stage of the manufacturing process, the diaper 1 is in a flat state stretching in the longitudinal direction, as shown in FIG.2. The diaper 1 in the flat state has a "longitudinal direction" and a "width direction" that are orthogonal to each other. The "longitudinal direction" is a direction conforming to the longitudinal direction of the absorbent main body 10, and is a direction corresponding to the vertical direction of the absorbent main body 10 in the underpants-shaped state (FIG. 1). The "width direction" is a direction corresponding to the lateral direction of the absorbent main body 10 in the underpants-shaped state. (FIG. 1)

In addition, in FIG. 2, the state of "stretching in the longitudinal direction" (the stretched state) means a state where, when stretching the diaper 1 in the longitudinal direction in resistance to the contractive force by elastic members (e.g., later-described waist elastic members 36) arranged extending along the longitudinal direction, the diaper is stretched to the extent that no wrinkles and gathers are seen in portions where any of the elastic members are arranged. Therefore, the shape of the diaper 1 in the state of being stretched in the longitudinal direction is identical to the shape of the diaper 1 that is flatly extended in the state where the contractive force by the elastic members does not exhibit.

Also, as shown in FIG. 3, the direction orthogonal to the longitudinal direction and the width direction is a "thickness direction". The side that comes into contact with the wearer's skin is the "skin side", and the opposite side is the "non-skin side".

The diaper 1 includes: the absorbent main body 10; a pair of the belt portions 30 and 30 that are joined to two lateral sides of the absorbent main body 10; and the joining portions 40 that join the absorbent main body 10 and the belt portions 30 and 30.

In the absorbent main body 10 in the stretched state shown in FIG. 2, the front end portion 10eLf and the back end portion 10eLb in the longitudinal direction each have a substantially V-shaped tapered shape. Further, the pair of belt portions 30 and 30, which are placed mainly around the wearer's waist, are arranged side-by-side in the width direction while being overlaid on the skin-side surface of the absorbent main body 10. The belt portions 30 are respectively joined by the joining portions 40 (40F, 40B) which are provided so as to be inclined along the V-shapes of the end portions 10eLf and 10eLb of the absorbent main body 10.

The absorbent main body 10 is folded one time at a center position CL10 in the longitudinal direction in a process in which, starting from the flat state of FIG. 2, while opening the widthwise inner end portions 30eW1 and 30eW1 of the pair of the belt portions 30 and 30 toward both widthwise sides, the belt portions 30 and 30 are each folded one time at a substantially center position CL30 in the longitudinal direction. Then, the waist opening BH of the diaper 1 is formed by the widthwise inner end portions 30eW1 of the belt portions 30 and the longitudinal end portions 10eLf and 10eLb of the absorbent main body 10. In addition, a pair of the leg openings LH are each formed by the widthwise outer end portion 30e1 of each belt portion 30 (outer edge of the belt portion 30) and the widthwise outer end portion 10e1 of the absorbent main body 10 (outer edge of the absorbent main body 10). Thereby, the diaper becomes in a state of being capable of putting on as an underpants-shaped diaper 1 as shown in FIG. 1.

### Absorbent main body 10

The absorbent main body 10 is a sheet-like member whose longitudinal end portions 10eLf and 10eLb are tapered into a substantially V-shape in plan view. The absorbent main body 10 includes: an absorbent core 11; a top sheet 12 that covers the absorbent core 11 from the skin side; a back film 13 that covers the absorbent core 11 from the non-skin side; a back sheet 14 that is overlaid on the non-skin side with respect to the back film 13; the side sheets 15 that are provided on the skin side of the back sheet 14, on both widthwise (lateral) sides of the absorbent main body 10; and leg elastic members 16 that stretch/contract in the longitudinal direction (see FIG. 3). These members are each connected to members which are adjacent thereto in thickness direction with a hot-melt adhesive, or the like. Note that examples of the application pattern of the adhesive include an Ω-shaped pattern, a spiral pattern, a stripe pattern, and the like. Furthermore, in addition to the joining means in which the adhesive is used, the members may be joined using welding means such as the heat welding and the ultrasonic welding, or other known joining means, for example.

The absorbent core 11 is a molded body obtained by molding a predetermined liquid absorbent material into a substantially oval shape in a plan view as shown in FIG. 2, as one example of a predetermined shape. Examples of the liquid absorbent material include the liquid-absorbent fiber (e.g., pulp fibers) and liquid-absorbent granules (e.g., superabsorbent polymers, so-called SAP). However, the shape of the absorbent core 11 is not limited to the oval shape as described above, and may be a substantially hourglass shape in a plan view or other shapes. Further, the outer circumferential surface of the absorbent core 11 may be covered with a core-wrapping sheet (not shown) such as tissue paper.

The top sheet 12 is a liquid-permeable sheet member such as the air-through nonwoven fabric sheet, for example, and is arranged so as to cover the absorbent core 11 from the skin side toward the non-skin side as shown in FIG. 3A. The longitudinal end portions of the top sheet 12 constitute the end portions 10eLf and 10eLb of the absorbent main body 10, and therefore they respectively have the aforementioned approximately V-shaped tapered shapes in which the dimensions in the width direction become smaller as it goes toward the front side or back side in the longitudinal direction.

The back film 13 is a liquid-impermeable leak-proof sheet such as polyethylene (PE) film, polypropylene (PP) film, or the like. The back sheet 14 is a sheet member made of flexible hydrophobic nonwoven fabric, etc., and is a member that constitutes the exterior of the absorbent main body 10. The back sheet 14, like the top sheet 12, has the aforementioned approximately V-shaped tapered shapes in which the dimensions in the width direction become smaller as it goes toward the front side or back side in the longitudinal direction.

The side sheet 15 is made of a flexible hydrophobic nonwoven fabric sheet, etc., like the back sheet 14, and is joined to the skin side of the back sheet 14 on both widthwise (lateral) sides. Between the side sheet 15 and the back sheet 14 in the thickness direction, a plurality of the leg elastic members 16 such as elastic strings are attached spacing in the width direction with being stretched in the longitudinal direction at a predetermined stretch factor. Based on the contractive force exhibited by the leg elastic members 16, the stretchability along the longitudinal direction is given to the widthwise outer end portions 10e1 and 10e1 of the absorbent main body 10, that is, the leg openings LH and LH. This improves the fit of the diaper 1 around the legs. Note that the member constituting the leg elastic member 16 is not limited to the above-mentioned elastic string, etc., and it is sufficient to be any member that can exhibit a contractive force along the longitudinal direction. For example, it may be the stretchable sheet member such as stretchable nonwoven fabric.

### Belt portion 30

The belt portions 30 are each a band-shaped member that connects the longitudinal front end portion 10eLf of the absorbent main body 10 (front upper end portion in the underpants-shaped state in FIG. 1) and the longitudinal back end portion 10eLb (back upper end portion in the pants-shaped state in FIG. 1). Each of the belt portions 30 includes a non-skin-side sheet 31, a skin side sheet 32, a waist elastic sheet 33, and the waist elastic members 36.

Both the non-skin-side sheet 31 and the skin-side sheet 32 are band-shaped sheet members arranged extending in the longitudinal direction, and is made of a spunbond nonwoven fabric, a SMS nonwoven fabric, etc., for example. In both longitudinal end portions of the non-skin-side sheet 31 and of the skin-side sheet 32, the dimensions in the width direction become narrower as it goes toward the front side or back side in the longitudinal direction, in accordance with the shape of both longitudinal end portions 10eLf and 10eLb of the absorbent main body 10. Further, the non-skin-side sheet 31 has a folded portion 31f in which a part of the non-skin-side sheet 31 is folded back outward at the widthwise (lateral) inner end portion 30eW.

The waist elastic sheet 33 and a plurality of the waist elastic members 36 are provided between the non-skin-side sheet 31 and the skin-side sheet 32 in the thickness direction. The waist elastic sheet 33 is the stretchable sheet member which has a stretchability along the longitudinal direction, and is made of stretchable nonwoven fabric and stretchable films, for example. In the present embodiment, the waist elastic sheet 33 is provided near the widthwise outer end portion 30e1 of the belt portion 30 (near a vertical lower end of the belt portion 30 in the pants-shaped state in FIG. 1), and the waist elastic sheet 33 gives an appropriate stretchability to the leg opening LH (outer end portion 30e1).

The plurality of waist elastic members 36 are the linear elastic member, such as the elastic string, which has the stretchability along the longitudinal direction, and are arranged spacing in the width direction. The waist elastic members 36 are provided near the widthwise inner end portion 30eW1 of the belt portion 30 (near a vertical upper end of the belt portion 30 in the underpants-shaped state in FIG. 1), and the waist elastic members 36 give an appropriate stretchability along the waist opening BH of the diaper 1. In particular, in the present embodiment, some of the waist elastic members 36 are placed overlaid on the folded portion 31f in the thickness direction, near the inner end portion 30eW1, increasing the density (see FIGS. 3A and 3B). This gives a sufficient stretching/contracting force to the waist opening BH, making it likely to prevent the diaper 1 from slipping off during usage. Further, some of the waist elastic members 36 are arranged overlapping with the waist elastic sheet 33.

### Joining portion 40

The joining portion 40 is a portion that connects the absorbent main body 10 and the belt portion 30. The joining portion 40 has a pair of the front joining portions 40F on the front side in the front-back direction and a pair of the back joining portions 40B on the back side. Specifically, in the front upper end portion 10eLf of the absorbent main body 10 (longitudinal front end portion in FIG. 2), a pair of the front joining portions 40F and 40F are provided to join the belt portions 30 and 30 onto both lateral sides. Similarly, the back upper end portion 10eLb (longitudinal back end portion in FIG. 2) a pair of the back joining portions 40B and 40B are provided to join the belt portions 30 and 30 onto both lateral sides. The front joining portion 40F and the back joining portion 40B are configured so as to incline from the waist opening BH toward the leg opening LH, from above to below in the vertical direction and from inside to outside in the lateral direction.

The joining portions 40 (the front joining portions 40F and the back joining portions 40B) provided in the diaper 1 of the present embodiment has a partially bent shape, as shown in FIGS. 1 and 2. That is, the front joining portions 40F each have a shape in which a vertical upper portion 40fa and a vertical lower portion 40fb are bent at a portion 40fp. In other words, the front joining portions 40F has a portion that protrudes inward in the lateral direction with respect to the straight line connecting the upper end 40fea and the lower end 40feb of the joining portion 40 (hereinafter also referred to as a convex portion 40fp). Similarly, the back joining portion 40B has a portion protrudes inward in the lateral direction with respect to the straight line connecting a vertical upper end 40bea and a vertical lower end 40beb (convex portion 40bp).

Although details will be described later, the joining portions 40 are formed by heat welding or ultrasonic welding, and join the absorbent main body 10 and the belt portion 30. However, the joining method by the joining portion 40 is not limited to this example, and may be a joining method of using an adhesive, other joining methods, or a combination thereof.

### Operation of putting on diaper 1

Next, the operation when putting on the diaper 1 will be described. Normally, when putting on an underpants-shaped disposable diaper like the diaper 1, the following operations are performed. First, each leg of the wearer is passed from the waist opening BH through a pair of the leg openings LH. Next, holding two lateral end portions of the belt portions 30, the diaper 1 is pulled up from the wearer's toe side to the crotch portion. Then, the absorbent main body 10 is made fit to the wearer's crotch portion, making the belt portion 30 fit around the wearer's waist.

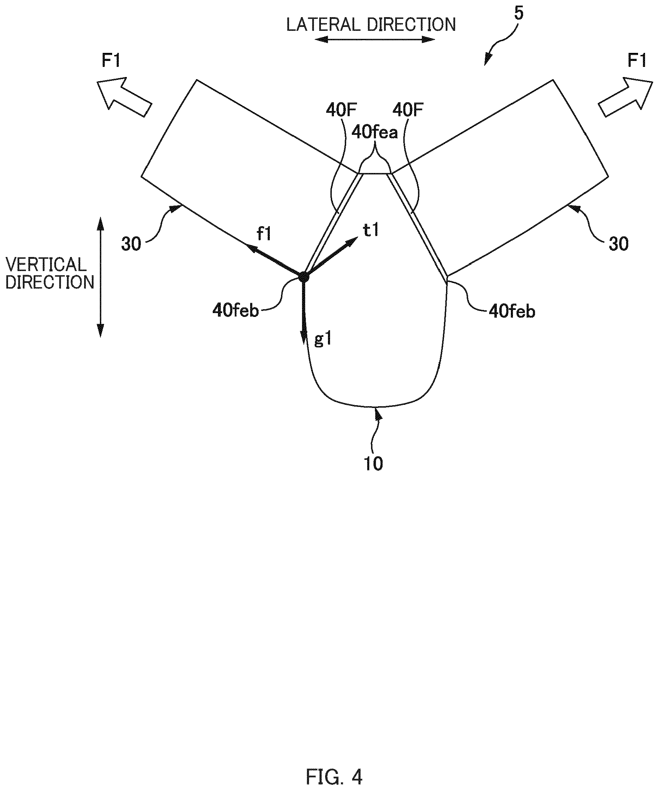

In such a putting-on operation, conventional underpants-shaped diapers cause a problem of breaking joining portions which join the absorbent main body and the belt portion. FIG. 4 is a diagram illustrating the relationship of forces acting on the joining portions 40 when putting on a conventional underpants-shaped diaper 5 as a comparative example. The basic configuration of the underpants-shaped diaper 5 (diaper 5) according to the comparative example is substantially the same as the diaper 1 according to the present embodiment, but the shape of the joining portions 40 is different. That is, the joining portions 40 of the diaper 5 are formed in a straight line extending from the vertical upper end 40fea to the vertical lower end 40feb, and do not have the convex portions 40fp like the diaper 1 between the upper end 40fea and the lower end 40feb.

When putting on the diaper 5 of the comparative example, as described above, after passing the wearer's legs through the diaper 5, the upper end portions of the pair of the belt portions 30 are held and pulled upward. That is, an operation is performed in which the waist opening BH, which is formed by the belt portions 30, is pulled upward in the vertical direction while spreading it outward in the lateral direction. At this time, the lateral outer end portions of the belt portions 30 are pulled by a diagonally-upward force F1 as shown in FIG. 4.

When the belt portions 30 is pulled diagonally upward by the force F1, a pulling force acts on the joining portions 40 (40F). In particular, a large force acts on the lower ends 40feb of the joining portions 40, which is far from the point of effort of the force F1. In the example of FIG. 4, a force f1 for pulling in the same direction as the force F1 (F1 > f1) acts on the lower end 40feb of the joining portion 40. In addition thereto, a vertically-downward drag force g1 acts on the lower end 40feb of the joining portion 40. Then, so as to balance these forces f1 and g1, a force t1 directed upward in the vertical direction and inward in the lateral direction acts on the lower end 40feb of the joining portion 40. In the operation of putting on the diaper 5, when the force t1 acting on the lower end 40feb of the joining portion 40 becomes large and has exceeded the joining strength of the joining portion 40, the joining portion 40 starts to tear from the lower end 40feb, and the tearing propagates along the joining portion 40 toward the upper end 40fea. This causes a risk that the belt portions 30 and the absorbent main body 10 are separated and a risk that the diaper 5 becomes disable to be put on the body.

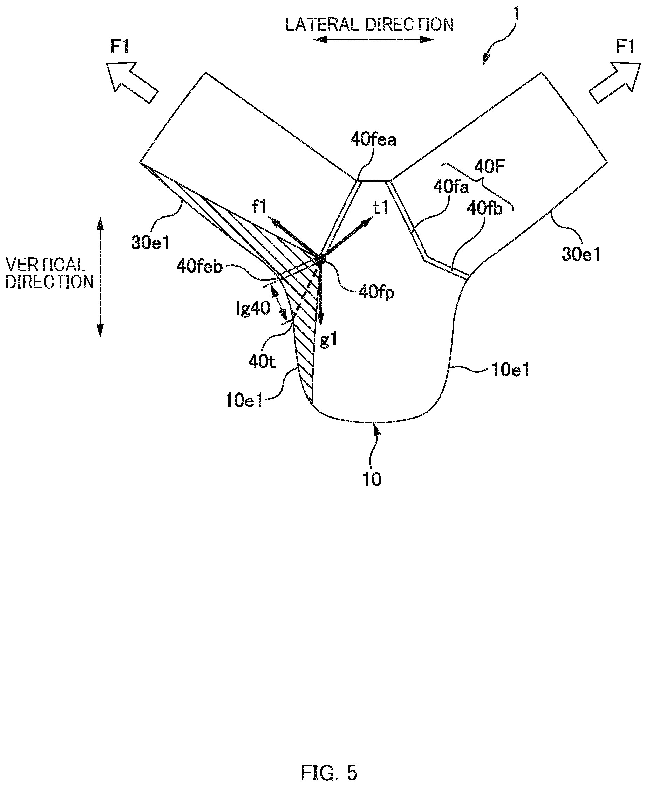

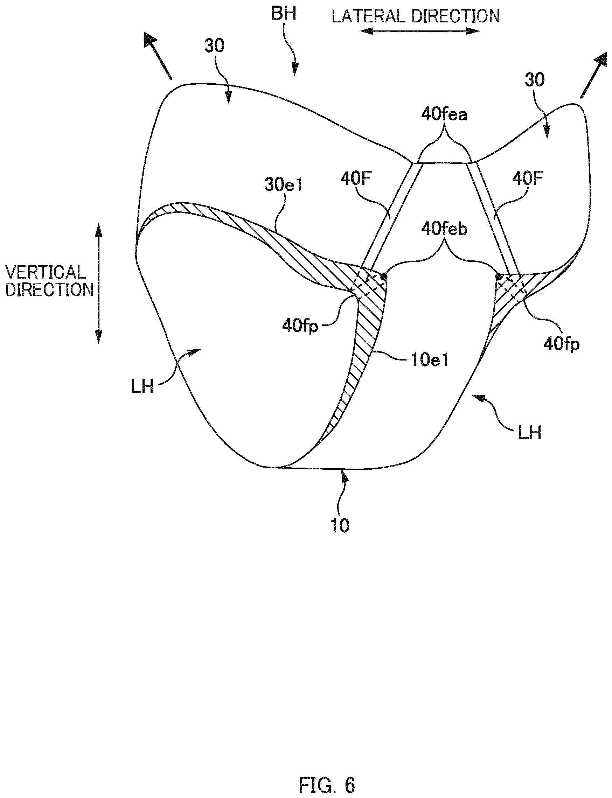

In contrast, in the case of the diaper 1 of the present embodiment, the force acting on the joining portion 40 during the putting-on operation is different from that of the diaper 5 of the comparative example. FIG. 5 is a diagram illustrating the relationship of forces acting on the joining portions 40 when putting on the underpants-shaped diaper 1 of the first embodiment. FIG. 6 is a perspective view showing the leg opening LH of the diaper 1 during the putting-on operation.

When putting on the diaper 1 of the present embodiment, similarly to the diaper 5, an operation is performed in which the waist opening BH, which is formed by the belt portions 30, is pulled upward in the vertical direction while spreading it outward in the lateral direction. At this time, the lateral outer end portion of the belt portions 30 are pulled by a diagonally upward force F1 (see FIG. 5).

As described above, the diaper 1 is provided with a convex portions 40fp that protrude inward in the lateral direction between the upper end 40fea and the lower end 40feb of the joining portion 40. By providing such convex portions 40fp, the circumference of the leg opening LH of the diaper 1 becomes longer than when the convex portion 40fp is not provided. For example, in FIG. 5, if the joining portion 40 is formed in a straight line, that is, if the joining portion 40 is formed as a straight line extending from the upper end 40fea through the convex portion 40fp to the imaginary lower end 40t, the position of the imaginary lower end 40t is located separated away from the lower end 40feb by a distance lg40. In other words, in the diaper 1 of the present embodiment, since the joining portion 40 is bent at the convex portion 40fp, the circumference of the outer end portion (outer edge) 10e1 of the absorbent main body 10 in the leg opening LH is larger by a length lg40 compared to the case where the joining portion 40 is not bent.

The outer end portion 10e1 of the absorbent main body 10 is a portion that comes into contact with the wearer's groin when putting on the diaper 1. Making longer the circumference of the outer end portion 10e1 results in a surplus of fabric that constitutes the outer end portion 10e1 (nonwoven fabric, etc.), and this makes it likely to form a gap between it and the wearer's skin (the groin). That is, the lower end 40feb of the joining portion 40 is made likely to rise up from the wearer's skin side to the non-skin side. As a result, the regions indicated by the hatched portions in FIG. 6 are rolled up from the convex portions 40fp of the joining portions 40 to the non-skin side, and the convex portions 40fp come into contact with the wearer's skin. This makes the lower ends 40feb not come into contact with the wearer's skin.

In this case, the point at which the force F1 that pulls up the belt portion 30 acts moves from the lower end 40feb of the joining portion 40 to the convex portion 40fp. Therefore, during putting on of the diaper 1, as shown in FIG.5, the following forces act on the convex portion 40fp of the joining portion 40: a force f1 for pulling in the same direction as the force F1; a vertically-downward drag force g1; and a force t1 directed upward in the vertical direction and inward in the lateral direction. The convex portion 40fp of the joining portion 40 is separated away from the lower end 40feb by a predetermined distance. That is, fabric that constitutes the absorbent main body 10 and the belt portion 30 (nonwoven fabric, etc.) exists around the convex portion 40fp, and therefore a starting point for tearing is less likely to generate even if the force (t1) is concentrated on the convex portion 40fp. In other words, even if the belt portions 30 are pulled up when putting on the diaper 1, the joining portion 40 is less likely to tear, and it suppresses separating of the belt portion 30 and the absorbent main body 10 and making the diaper 1 unable to wear.

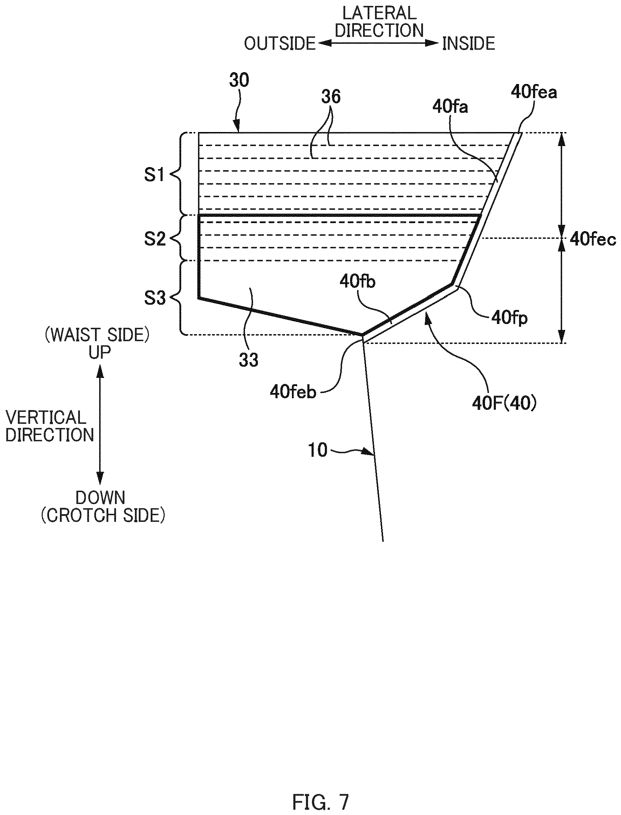

FIG. 7 is a diagram illustrating the arrangement of the convex portion 40fp provided in the joining portion 40. FIG. 7 shows the planar shapes of the belt portion 30 and the joining portion 40 when the diaper 1 in the underpants-shaped state is stretched in the vertical direction and in the lateral direction.

As shown in FIG. 7, in the vertical direction, the convex portion 40fp is provided below the intermediate position 40fec between the upper end 40fea and the lower end 40feb of the joining portion 40. As described in FIGS. 5 and 6, in the case where the convex portion 40fp is provided in the joining portion 40, during usage of the diaper 1, a region between the convex portion 40fp and the lower end 40feb is likely to rise up from the wearer's skin side to the non-skin side. Therefore, in the case where the convex portion 40fp of the joining portion 40 is located above the intermediate position 40fec in the vertical direction, the area of a portion where the belt portion 30 rises up from the wearer's skin will increase, making it likely to impair the fit around the wearer's legs. In contrast, in the diaper 1, since the convex portion 40fp is provided below the intermediate position 40fec of the joining portion 40 in the vertical direction as shown in FIG. 7, the area where the belt portion 30 rises up from the wearer's skin when putting on the diaper 1 decreases compared to the case where the convex portion 40fp is provided above the intermediate position 40fec. Therefore, it is possible to suppress the deterioration of the fit around the wearer's legs. In addition, in the case where the convex portion 40fp is placed below the intermediate position 40fec, it is less likely to suppress the contractive force that acts near the upper end portions of the belt portions 30 by the waist elastic member 36. This makes it likely to maintain the fit in the waist opening BH, and makes it possible to prevent the belt portions 30 from slipping down.

In addition, in the belt portion 30 of diaper 1, the number of the sheet members that are stacked in the thickness direction in the vertical lower end portion is greater than the number of the sheet members that are stacked in the thickness direction in the remaining portions (the vertical upper end portion, etc.). As described above, in the belt portion 30, the non-skin-side sheet 31 and the skin-side sheet 32 are stacked, and in a partial region (vertical lower end portion), the waist elastic sheet 33 is interposed between and stacked with the non-skin-side sheet 31 and skin-side sheet 32 (see FIG. 3A, etc.). In other words, at least a part of the lower end portion of the belt portion 30 is a multi-stacked region (region surrounded by a thick line in FIG. 7), in which the number of stacked sheets is greater than in the upper end portion by the number of sheets of the waist elastic sheet 33. In the multi-stacked region, when joining the belt portion 30 and the absorbent main body 10 by the joining portion 40, the number of joining material is larger that in a remaining regions, and this can make it likely to increase the welding strength. Therefore, when putting on the diaper 1, the joining portion 40 is less likely to tear in the multi-stacked region (lower end portion) of the belt portion 30 against the force of pulling the belt portion 30 diagonally upward, and this makes it likely to suppress the belt portion 30 and the absorbent main body 10 from separating.

In the diaper 1, the convex portion 40fp of the joining portion 40 is provided in the multi-stacked region (see FIG. 7). The convex portion 40fp of the joining portion 40 is a portion that protrudes the farthest inwardly in the lateral direction, and is a portion on which the largest force is applied when pulling up the belt portion 30. Therefore, in the belt portion 30, by arranging the convex portion 40fp in the multi-stacked region, in which the number of stacked sheets is larger and the stiffness is high compared to the remaining regions, it is possible to make the joining portion 40 less likely to tear even in the case where a large force acts on the convex portion 40fp.

In addition, in the above case, the basis weight of the sheet members which constitute the belt portion 30 is higher in the vertical lower end portion than in the remaining portions (vertical upper end portion, etc.). In other words, at least a part of the lower end portion of the belt portion 30 is a high basis-weight region, in which the basis weight is higher than in the upper end portion due to the presence of the waist elastic sheet 33. In the high basis-weight region, when joining the belt portion 30 and the absorbent main body 10 by the joining portion 40, the number of joining material is larger that in a remaining regions, and this can make it likely to increase the welding strength. Therefore, when putting on the diaper 1, the joining portion 40 is less likely to tear in the high basis-weight region (lower end portion) of the belt portion 30 against the force of pulling the belt portion 30 diagonally upward, and this makes it likely to suppress the belt portion 30 and the absorbent main body 10 from separating. In addition, as in the above case, by providing the convex portion 40fp of the joining portion 40 in the high basis-weight region, it is possible to make the joining portion 40 less likely to tear even in the case where a large force acts on the convex portion 40fp.

In addition, regarding regions of the belt portion 30 where the elastic members are provided, letting a region where only the waist elastic members 36 (linear elastic member) such as the elastic strings are arranged be a region S1, letting a region where only the waist elastic sheet 33 (stretchable sheet member) made of stretchable nonwoven fabric and the like is arranged be a region S3, and letting a region where the waist elastic members 36 and the waist elastic sheet 33 are arranged overlapped be a region S2, it is desirable that the convex portion 40fp of the joining portion 40 is provided below the region S2 (see FIG. 7). In the region S2, the contractive force of both of the waist elastic members 36 and the waist elastic sheet 33 acts, and therefore the region S2 is a region where the stress is strongest when pulling up the belt portion 30 during the putting-on operation of the diaper 1. Therefore, if the convex portion 40fp is provided in this region S2, there is a risk that a large force acts on the convex portion 40fp, making the joining portion 40 likely to tear. In contrast, in the region S3 below the region S2, excessively large stress is less likely to act compared to the region S2, and therefore providing the convex portion 40fp in the region S3 suppresses that a large force acts on the convex portion 40fp. In addition, the stretching/contracting force by the waist elastic members 36 provided in the region S1 are less likely to interfere with the convex portion 40fp, and therefore the fit of the waist opening BH is less likely to deteriorate in the upper end portion of the belt portion 30.

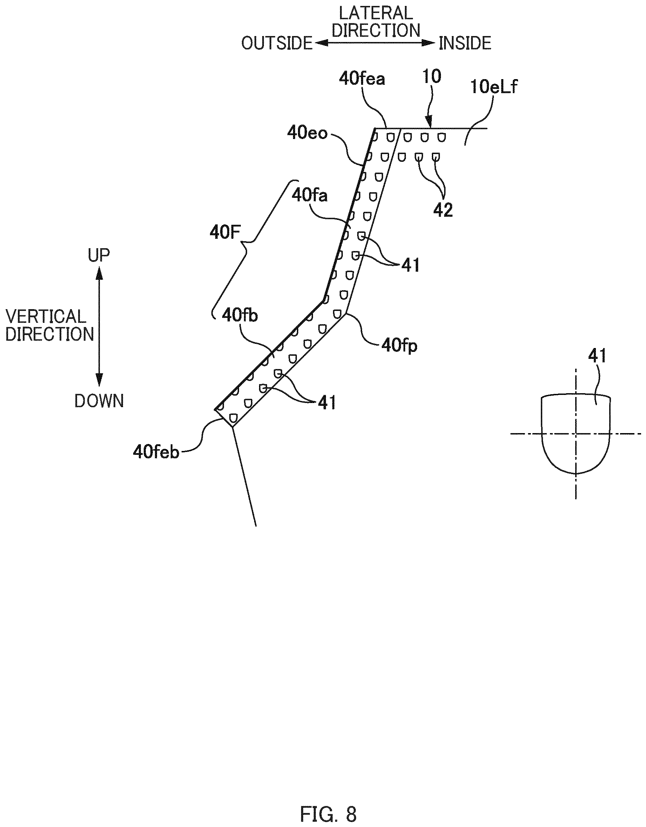

FIG. 8 is an enlarged plan view illustrating the structure of the joining portion 40. In the diaper 1 of the present embodiment, a plurality of the sealing portions 41 formed by ultrasonic welding and heat welding are arranged in a discrete manner in a predetermined region, thereby forming the joining portion 40. By arranging the plurality of small sealing portions 41 at intervals, the force acting on the joining portion 40 (e.g., force t1 in FIG. 5) is distributed and is less likely to concentrate in one location. This makes it less likely to tear the joining portion 40.

Further, it is desirable that the shape of the sealing portions 41 is such that its lower side in the vertical direction is curved. Specifically, as shown in FIG. 8, it is desirable that the outline of the sealing portion 41 is curved below the center position of the sealing portion 41 in the vertical direction. During the putting-on operation of the diaper 1, a force directed from below to above in the vertical direction and from the one side to the other side in the lateral direction, as represented by the force t1 in FIG. 5, acts on the joining portion 40 (the sealing portion 41). At this time, if the lower outline of the sealing portion 41 is straight and a "corner" is formed therein, there is a risk that the diagonally-upward force (t1) acting on the sealing portion 41 concentrates on such a "corner", making the sealing portion 41 likely to peel off. In contrast, if a "corner" is not formed due to making the lower outline of the sealing portion 41 curved, the force (t1) acting on the sealing portion 41 is less likely to concentrate in one location, making the sealing portion 41 less likely to peel off. This makes the joining portion 40 less likely to tear.

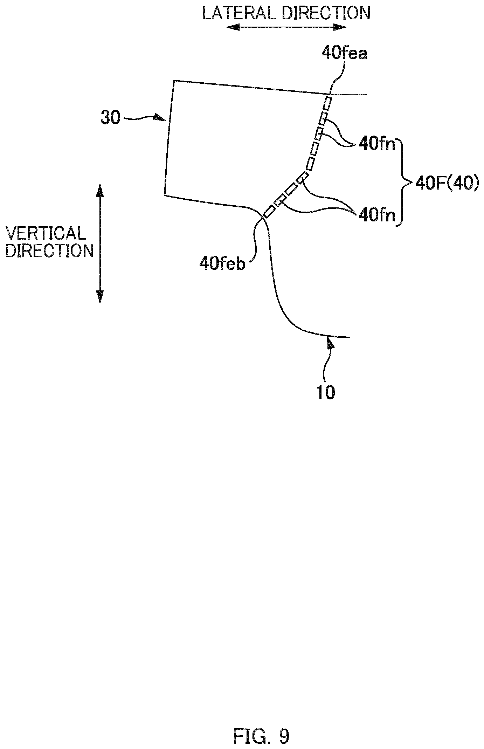

Further, the discontinuous portions may be provided in the joining portion 40. FIG. 9 is a plan view illustrating a case where the discontinuous portions are provided in the joining portion 40. The joining portion 40F (40) shown in FIG. 9 is formed in a substantially dotted-line shape in which a plurality of the discontinuous portions are formed between the upper end 40fea and the lower end 40feb. In other words, the joining portion 40F is formed by a plurality of strip-shaped, short joining portions 40fn being lined up intermittently. For example, among the plurality of joining portions 40fn constituting the joining portion 40F, a certain one is defined as a joining portion 40fn1, and another one that is adjacent to the joining portion 40fn1 at a distance is defined as a joining portion 40fn2. At this time, even if tearing occurs in the certain joining portion 40fn1, since there is a gap (discontinuous portion) between it and the adjacent joining portion 40fn2, it suppresses the immediate propagation of the tearing from the joining portion 40fn1 to the joining portion 40fn2. That is, since the effect of the tearing is limited to only the certain joining portion 40fn1, it can make it likely to prevent the entirety of the joining portion 40 from tearing.

Note that the discontinuous portions provided in the joining portion 40 may be a gap between two adjacent sealing portions 41 and 41 in FIG. 8. That is, a plurality of the sealing portions 41, 41,... may be arranged in a discrete manner with a certain distance between them.

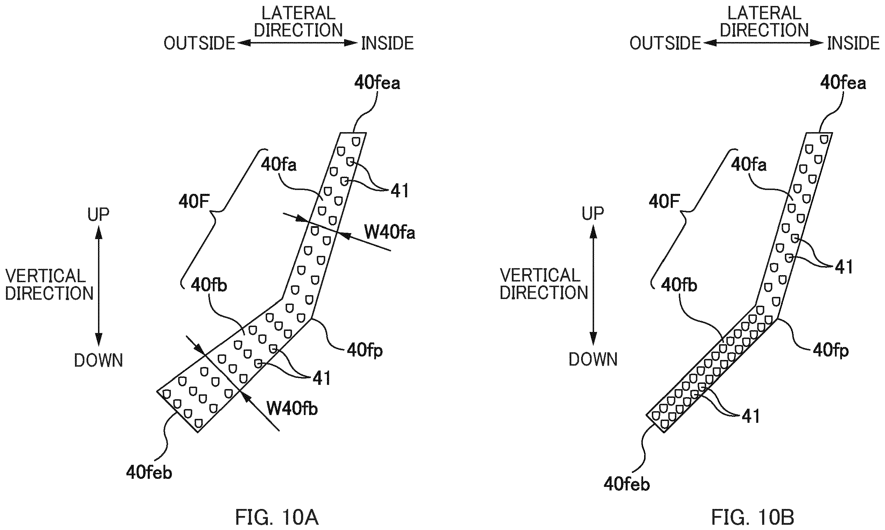

Further, the arrangement of the sealing portions 41 that constitute the joining portion 40 can also be modified as follows. FIGS. 10A and 10B are diagrams showing modified examples of the arrangement of the sealing portions 41 that constitute the joining portion 40.

In FIG. 10A, the width of the joining portion 40 becomes thicker toward the lower portion. That is, the width W40fb of the portion 40fb that is located below the convex portion 40fp of the joining portion 40 is thicker than the width W40fa of the portion 40fa that is located above the convex portion 40fp (W40fb > W40fa). The joining portion 40 thus has a portion where the width W40fb on the lower portion (40fb) is thicker than the width W40fa on the upper portion (40fa), and therefore, when pulling up the belt portion 30 during the operation of putting on the diaper 1, the force (t1) that acts on the joining portion 40 from below to above in the vertical direction can be received by the wide portion in the lower portion (40fb) of the joining portion 40. Therefore, the force that acts on the lower portion (40fb) of the joining portion 40 is likely to be distributed in the width direction, and it can make it likely to prevent the joining portion 40 from tearing.

On the other hand, in FIG. 10B, the density of the sealing portions 41, which form the joining portion 40, increases toward the lower portion of the joining portion 40. In other words, the area of the sealing portions 41 provided per unit area in the portion 40fb of the joining portion 40 that is located below the convex portion 40fp is larger than the area of the sealing portions 41 provided per unit area in the portion 40fa of the joining portion 40 that is located above the convex portion 40fp. In this way, the joining portion 40 has a portion where the density of the sealing portions 41 in the lower portion (40fb) is larger than the density of the sealing portions 41 on the upper portion (40fa), and therefore the welding strength in the lower portion (40fb) of the joining portion 40 is higher than the welding strength in the upper portion (40fa) of the joining portion 40. Therefore, when pulling up the belt portion 30 during the operation of putting on the diaper 1, it makes it likely to resist the force (t1) that acts on the joining portion 40 from below to above in the vertical direction. Thereby, even if a force acts on the lower portion (40fb) of the joining portion 40, it is possible to easily prevent the joining portion 40 from tearing.

Further, the joining portion 40 has a joined portion and an unjoined portion, in the end (edge) located on the non-skin side in the thickness direction. Note that the non-skin-side ends (edges) of the joining portions 40 refer to the tip-end portions of the joining portions 40F and 40B that protrude from the skin side to the non-skin side in the cross-sectional view of the waist opening BH in FIG. 1. In FIG. 8, at least some of the plurality of sealing portions 41 that form the joining portion 40 are arranged overlapping with the non-skin-side end of the joining portion 40 (end 40eo indicated by a thick line in FIG. 8). That is, at least a part of the non-skin-side end 40eo of the joining portion 40 is joined by the sealing portions 41. If the sealing portions 41 are formed over the entirety of the non-skin-side end 40eo of the joining portion 40, the stiffness of the end 40eo increases, and there is a risk that, when the wearer touches the end 40eo at the time of putting on the diaper 1, the discomfort is likely to occur. On the other hand, if no sealing portion 41 is formed over the entirety of the non-skin-side end 40eo, the joining between the absorbent main body 10 and the belt portions 30 at the end 40eo is likely to separate due to friction between the diaper 1 and the wearer's clothes, and the like. In contrast, in the diaper 1 of the present embodiment, by providing a joined portion and an unjoined portion in the non-skin-side end 40eo of the joining portion 40, it makes discomfort less likely to occur when the wearer touches the joining portion 40 and makes it possible to prevent the joining portion 40 from peeling off.

Furthermore, in the diaper 1, in addition to the sealing portions 41 that form the joining portions 40 that join the absorbent main body 10 and the belt portion 30, there may be provided the absorbent-main-body sealing portions 42 that join only the sheet members that form the absorbent main body 10. In FIG. 8, in at least a part of the front upper end portion 10eLf of the absorbent main body 10, a plurality of the absorbent-main-body sealing portions 42 are provided that join in the thickness direction the top sheet 12 and the back sheet 14 (back film 13) both of which constitute the absorbent main body 10. Due to the presence of the absorbent-main-body sealing portions 42 in the front upper end portion 10eLf of the absorbent main body 10, it is possible to suppress fraying between sheet members (the top sheet 12 and the back sheet 14), and to suppress the opening of the absorbent main body 10 in the upper end 10eLf.

Note that the sealing portions 41 forming the joining portions 40 and the absorbent-main-body sealing portion 42 established in the upper end portion of the absorbent main body 10 can be formed at the same time in the manufacturing process of the diaper 1. For example, in the diaper 1 that is in the stretched state as shown in FIG. 2, simultaneously when forming in portions where the joining portions 40 are formed the sealing portions 41 where the absorbent main body 10 and the belt portion 30 are welded in the thickness direction, the absorbent-main-body sealing portions 42 where the sheet members (12, 14) that constitute the absorbent main body 10 are welded in the thickness direction can be formed in the upper end portions 10eLf and 10eLb of the absorbent main body 10.

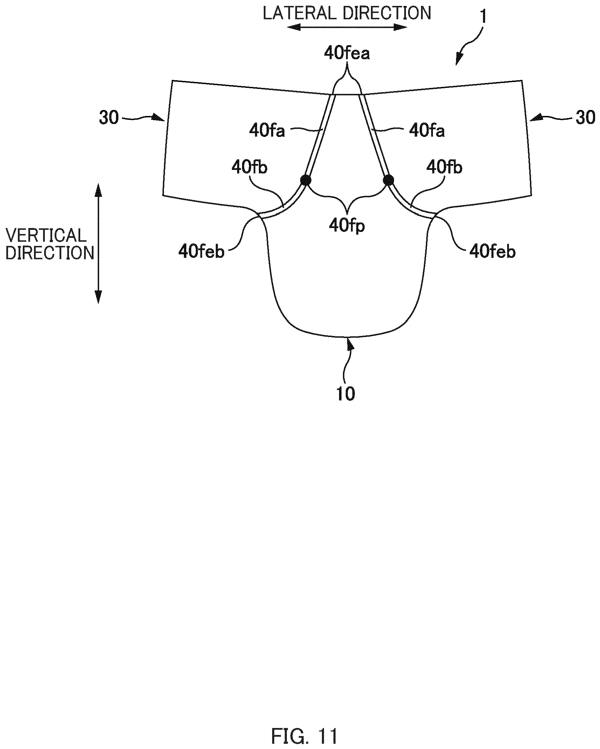

FIG. 11 is a schematic plan view illustrating a modified example of the joining portions 40. In the above-mentioned embodiment, the portion 40fa of the joining portion 40 located on the upper side in the vertical direction and the portion 40fb located on the lower side in the vertical direction both have a linear shape, and the convex portion 40fp have a shape protruding inward in the lateral direction. However, a part of the joining portion 40 may be formed in a curved shape as shown in FIG. 11. In FIG. 11, the lower portion 40fb of the joining portion 40 in the vertical direction is formed in an arc shape protruding inward in the lateral direction, and is smoothly connected to the upper portion 40fa at a convex portion 40fp. Even with such a configuration, as illustrated in FIGS. 5 and 6, the force that pulls up the belt portions 30 during the operation of putting on the diaper 1 is likely to act on the convex portion 40fp instead of the lower end 40feb. This can make the joining portion 40 less likely to tear compared to conventional underpants-shaped diapers. In addition, the upper portion 40fa and the lower portion 40fb of the joining portion 40 are continuously connected at the convex portion 40fp, and no corner is formed in the lower portion 40fb, making force concentration less likely to occur in the portion. Furthermore, since the line connecting the convex portion 40fp and the lower end 40feb of the joining portion 40 is curved, the length of the joining portion 40 (lower portion 40fb) itself becomes longer compared to the case where the convex portion 40fp and the lower end 40feb are connected in a straight line at the minimum distance. These make more likely to distribute the force acting on the joining portion 40, and can make it more likely to prevent the joining portion 40 from tearing.

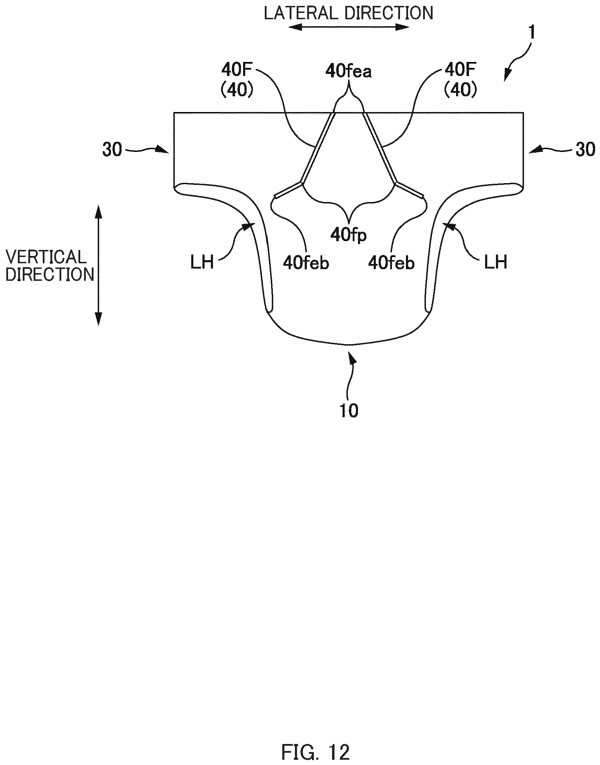

FIG. 12 is a schematic plan view illustrating another modified example of the joining portions 40. In FIG. 12, below the lower end 40feb of the joining portion 40, there is provided a portion where the belt portion 30 and the absorbent main body 10 are continuous. In other words, the lower end 40feb of the joining portion 40 does not reach the leg opening LH, and there is a part of the base material (nonwoven fabric, etc.) that constitutes the belt portion 30 and the absorbent main body 10, between the lower end 40feb and the leg opening LH. Therefore, even if the belt portion 30 is pulled up during the operation of putting on the diaper 1, it is likely to suppress the formation of a tear starting point in the lower end 40feb. This can makes the joining portion 40 further less likely to tear.

### Second embodiment

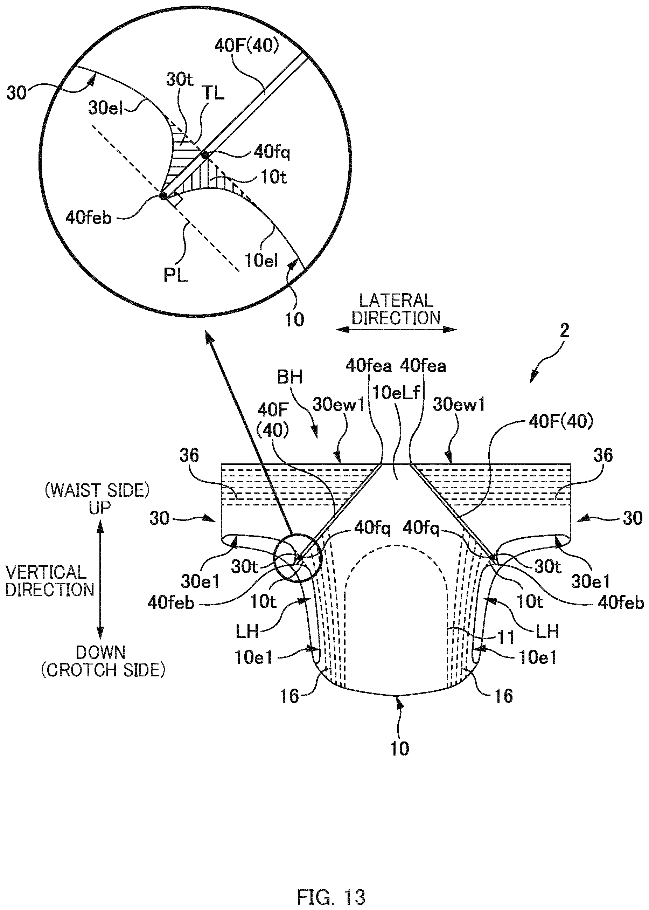

In the second embodiment, the following describes an underpants-shaped disposable diaper 2 (hereinafter also referred to as "diaper 2") having a different structure around the lower end portions of the joining portions 40 (40F, 40B) that join the absorbent main body 10 and the belt portions 30. FIG. 13 is a schematic front view of the diaper 2 of the second embodiment as seen from the front side.

As shown in FIG. 13, the diaper 2 of the second embodiment has a portion where a part of the absorbent main body 10 and a part of the belt portion 30 protrude toward the leg opening LH, in a predetermined region including the lower end 40feb of the front joining portion 40F. Specifically, the absorbent main body 10 has a projected portion 10t that protrudes toward the leg opening LH side, and the belt portion 30 has a projected portion 30t that protrudes toward the leg opening LH side (both are hatched portions in a partially enlarged view of FIG. 13). Similarly, regarding the back joining portion 40B, in the lower end 40beb, the absorbent main body 10 and the belt portion 30 respectively have portions that protrude toward the leg opening LH side (not shown in FIG. 13).

The projected portion 10t and the projected portion 30t can be specified as follows. First, draw a line (referred to as auxiliary line PL) that passes through the lower end 40feb of the front joining portion 40F and is perpendicular to the front joining portion 40F. Next, draw a tangent (referred to as a common tangent TL) that is common to the outer end portion 10e1 of the absorbent main body 10 (outline of the leg opening LH formed by the absorbent main body 10) and the outer end portion 30e1 of the belt portion 30 (outline of the leg opening LH formed by the belt portion 30). Then, a portion where the absorbent main body 10 exists and a portion where the absorbent main body 10 does not exist are formed in a region between the auxiliary line PL and the common tangent TL. Then, the portion where the absorbent main body 10 exists and that is located between the auxiliary line PL and the common tangent TL is the projected portion 10t. Similarly, in the region between the auxiliary line PL and the common tangent TL, a portion where the belt portion 30 exists and a portion where the belt portion 30 does not exist are formed, and the portion where the belt portion 30 exists is the projected portion 30t.

In addition, in FIG. 13, the intersection of the common tangent TL and the front joining portion 40F is defined as a midway point 40fq. That is, the midway point 40fq is a point located between the upper end 40fea and the lower end 40feb of the front joining portion 40F. Further, in FIG. 13, the joining portion 40 is formed in a straight line, but similarly to the diaper 1 of the first embodiment, the joining portion 40 may be bent or curved between the upper end 40fea and the lower end 40feb. Regarding diaper 2, the basic configuration other than the above (e.g., the configuration of the absorbent main body 10 and the belt portion 30, the configuration of the sealing portions 41 that form the joining portion 40, etc.) is substantially the same as diaper 1 described in the first embodiment, and therefore the detailed description thereof will be omitted.

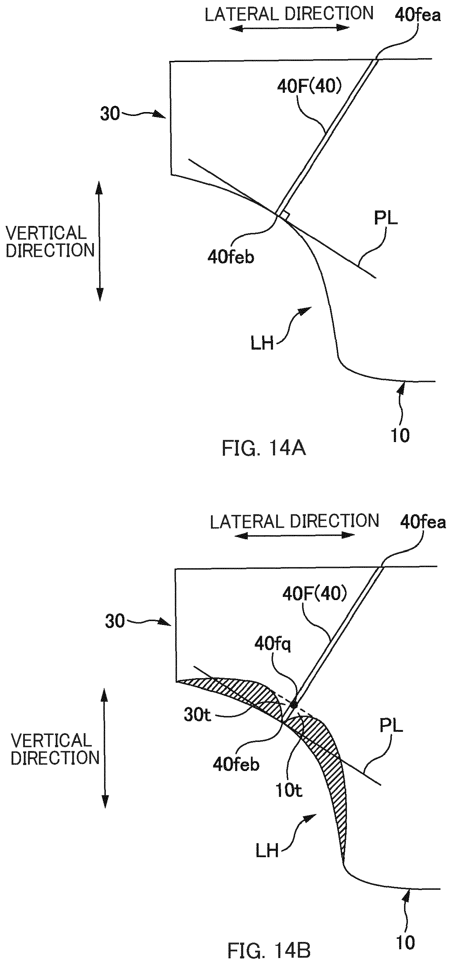

FIGS. 14A and 14B are diagrams illustrating an example of a method for forming the projected portion 10t and the projected portion 30t. For example, the projected portion 10t and the projected portion 30t can be formed by cutting out (cutting) a part of the absorbent main body 10 and a part of the belt portion 30 in a region that is located on the front joining portion 40F side with respect to the auxiliary line PL in the manufacturing process of the diaper 2.

FIG. 14A shows a part of the diaper 2 in a state before the projected portion 10t and the projected portion 30t are formed in the lower end 40feb of the front joining portion 40F. In the state shown in FIG. 14A, the sheet members which constitute the absorbent main body 10 and the belt portion 30 exist in the entire region on the front joining portion 40F side with respect to the auxiliary line PL passing through the lower end 40feb. In other words, on the front joining portion 40F side with respect to the auxiliary line PL, there is no region where the absorbent main body 10 and the belt portion 30 do not exist.

In this state, of the absorbent main body 10 and the belt portion 30, a part of regions extending along the leg opening LH is cut out (cut). In FIG. 14B, of the absorbent main body 10 and the belt portion 30, the hatched regions are cut out. As a result, portions where the absorbent main body 10 and the belt portion 30 do not exist (the hatched portions in FIG. 14B) are formed on the front joining portion 40F side with respect to the auxiliary line PL. Of the absorbent main body 10 and the belt portion 30, the remaining portions that have not been cut out become the projected portion 10t and the projected portion 30t. However, the method of forming the projected portion 10t and the projected portion 30t is not limited to this.

In addition, in the diaper 2, only either one of the projected portion 10t and the projected portion 30t may be formed. For example, only either one of the projected portion 10t and the projected portion 30t may be formed by cutting out only either one of the absorbent main body 10 and the belt portion 30 on the front joining portion 40F side with respect to the auxiliary line PL.

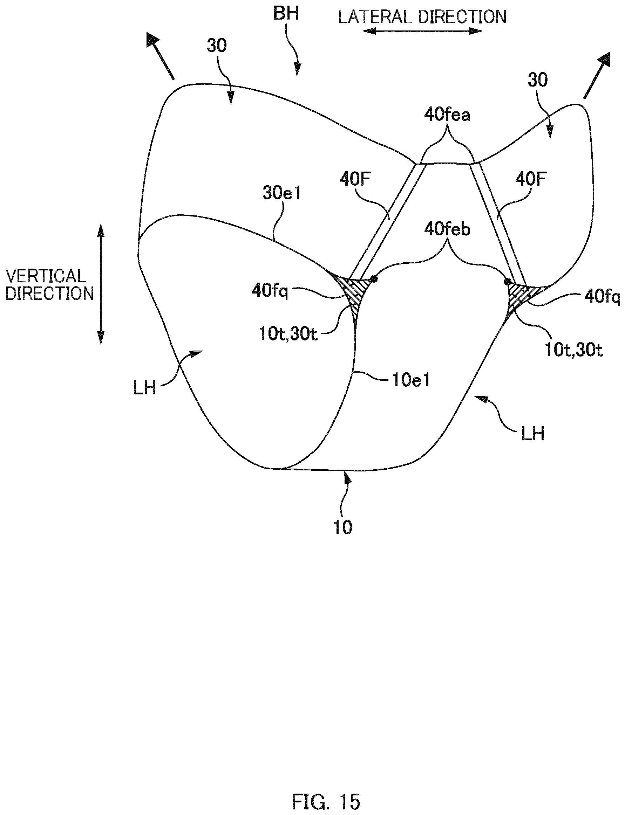

When putting on the diaper 2, operations similar to those for the diaper 1 are performed. That is, with the wearer's legs passed through the leg openings LH, an operation is performed in which the upper ends of the belt portions 30 on both lateral sides are held and pulled diagonally upward. FIG. 15 is a perspective view showing the leg opening LH of the diaper 2 during the putting-on operation.

In diaper 2, since the projected portion 10t of the absorbent main body 10 and the projected portion 30t of the belt portion 30 are provided, the circumference of the leg opening LH is longer compared to the case where the projected portion 10t and the projected portion 30t are not provided. Therefore, in the leg opening LH, the projected portion 10t and the projected portion 30t have a surplus of fabric, and this makes it likely to form a gap between it and the wearer's skin (the groin). In other words, the projected portion 10t and the projected portion 30t is made likely to rise up from the wearer's skin side to the non-skin side. As a result, the regions indicated by the hatched portions in FIG. 15 (projected portions 10t and 30t) are rolled up from the midway point 40fq to the non-skin side, and the midway point 40fq come into contact with the wearer's skin. This makes the lower ends 40feb not come into contact with the wearer's skin.

At this time, the point at which the force that pulls up the belt portion 30 acts shifts from the lower end 40feb of the joining portion 40 to the midway point 40fq. In other words, the midway point 40fq functions in the same way as the convex portion 40fp in the diaper 1 of the first embodiment, and a force is likely to concentrate and act on the midway point 40fq. the projected portion 10t and the projected portion 30t are Located between the midway point 40fq and lower end 40feb of the joining portion 40, and therefore even if a force acts on the midway point 40fq, the force is less likely to act on the lower end 40feb, making the starting point of a tear less likely to be formed. This makes the joining portion 40 (40F, 40B) less likely to tear even when pulling up the belt portion 30 at the time of putting on the diaper 2, and this prevents the separation of the belt portion 30 and the absorbent main body 10, and prevents the diaper 2 from becoming disable to be put on.

### Other embodiments

Although the embodiments of the present disclosure have been described hereinabove, the above embodiments of the present disclosure are simply to facilitate understanding of the present disclosure and are not in any way to be construed as limiting the present disclosure. The present disclosure may variously be changed or altered without departing from its gist and encompass equivalents thereof. For example, the following modifications are possible.

In the above-mentioned the embodiment, the underpants-shaped diaper 1 is described as an example of the underpants-shaped absorbent article, but the present invention is also applicable to other underpants-shaped absorbent article except for underpants-shaped diapers. For example, it may be an underpants-shaped sanitary napkin (shorts-type sanitary napkin) for children or adults, an underpants-shaped (shorts-type) absorbent pad, and the like.

### REFERENCE SIGNS LIST

1 diaper (underpants-shaped absorbent article, first embodiment),
2 diaper (underpants-shaped absorbent article, second embodiment),
5 diaper (underpants-shaped absorbent article, comparative example),
10 absorbent main body,
10eLf end portion, 10eLb end portion, 10e1 outer end portion,
10t projected portion,
11 absorbent core,
12 top sheet, 13 back film, 14 back sheet, 15 side sheet, 16 leg elastic member,
30. the belt portion,
30eW1 inner end portion, 30e1 outer end portion,
30t the projected portion,
31 non-skin-side sheet, 31f folded portion, 32 skin side sheet,
33 waist elastic sheet, 36 waist elastic member,
40. joining portion,
40F front joining portion,
40fa portion (upper side), 40fea upper end,
40fb portion (lower side), 40feb lower end,
40fp convex portion, 40fq midway point,
40fec intermediate position,
40B back joining portion,
40ba portion (upper side), 40bea upper end,
40bb portion (lower side), 40beb lower end,
40bp convex portion,
41. sealing portion, 42 absorbent-main-body sealing portion,
BH waist opening, LH leg opening,
CL10 center position (absorbent main body), CL30 center position (belt portion)

## Claims

1. An underpants-shaped absorbent article having a vertical direction, a lateral direction, and a front-back direction that intersect with each other,
the underpants-shaped absorbent article comprising:
an absorbent main body; and
a belt portion that connects a front upper end portion and a back upper end portion of the absorbent main body,
the underpants-shaped absorbent article having a waist opening and a pair of leg openings,
the belt portion being joined to the absorbent main body by a joining portion that inclines from the waist opening towards the leg opening from above to below in the vertical direction and from inside to outside in the lateral direction,
when the underpants-shaped absorbent article in a stretched state is viewed along the front-back direction,
the joining portion having a portion that protrudes inward in the lateral direction with respect to a straight line that connects a vertical upper end and a vertical lower end of the joining portion.

2. The underpants-shaped absorbent article according to claim 1, wherein
the joining portion has the portion that protrudes inward in the lateral direction,
the portion being located in the vertical direction below an intermediate position between an upper end of the joining portion and a lower end of the joining portion.

3. The underpants-shaped absorbent article according to claim 1 or 2, wherein
of the joining portion, at least a part of the portion that protrudes inward in the lateral direction is curved.

4. The underpants-shaped absorbent article according to any one of claims 1 to 3, wherein
the joining portion includes a plurality of sealing portions that are arranged in a discrete manner, and
at least one of the plurality of sealing portions has a curved outline, below a center position of the at least one of the plurality of sealing portions in the vertical direction.

5. The underpants-shaped absorbent article according to any one of claims 1 to 4, wherein
the joining portion has a discontinuous portion between the vertical upper end and the vertical lower end.

6. The underpants-shaped absorbent article according to any one of claims 1 to 5, wherein
the joining portion has a portion where a width in a region located vertically below the portion that protrudes inward in the lateral direction is greater than a width in a region located vertically above the portion that protrudes inward in the lateral direction.

7. The underpants-shaped absorbent article according to any one of claims 1 to 6, wherein
the joining portion includes a plurality of sealing portions that are arranged in a discrete manner, and
concerning a density per unit area of the sealing portion,
the joining portion has a portion where the density in a region located vertically below the portion that protrudes inward in the lateral direction is greater than the density in a region located vertically above the portion that protrudes inward in the lateral direction.

8. The underpants-shaped absorbent article according to any one of claims 1 to 7, wherein
the joining portion has a joined portion and an unjoined portion, in an end located on a non-skin side.

9. The underpants-shaped absorbent article according to any one of claims 1 to 8, wherein
in at least a part of an upper end portion of the absorbent main body,
the underpants-shaped absorbent article has an absorbent-main-body sealing portion that joins only a sheet member that constitutes the absorbent main body.

10. The underpants-shaped absorbent article according to any one of claims 1 to 9, wherein
a high basis-weight region is provided in at least a part of a lower end portion of the belt portion,
the high basis-weight region being a region where a basis weight is higher than in a remaining portion.

11. The underpants-shaped absorbent article according to any one of claims 1 to 9, wherein
a multi-stacked region is provided in at least a part of a lower end portion of the belt portion,
the multi-stacked region being a region where a number of sheet members that are stacked and constitute the belt portion is larger than in a remaining portion.

12. The underpants-shaped absorbent article according to claim 11, wherein
in the multi-stacked region, the joining portion has a portion that protrudes inward in the lateral direction.

13. The underpants-shaped absorbent article according to any one of claims 1 to 12, wherein
the belt portion has a stretchable sheet member and a linear elastic member both of which stretch and contract in the lateral direction, and
the joining portion has a portion that protrudes inward in the lateral direction, vertically below a region of the belt portion where the stretchable sheet member and the linear elastic member are stacked.

14. The underpants-shaped absorbent article according to any one of claims 1 to 13, wherein
in a region located in the vertical direction below the lower end of the joining portion,
the underpants-shaped absorbent article has a portion where the belt portion and the absorbent main body are continuous.

15. An underpants-shaped absorbent article having a vertical direction, a lateral direction, and a front-back direction that intersect with each other,
the underpants-shaped absorbent article comprising:
an absorbent main body; and
a belt portion that connects a front upper end portion and a back upper end portion of the absorbent main body,
the underpants-shaped absorbent article having a waist opening and a pair of leg openings,
the belt portion being joined to the absorbent main body by a joining portion that inclines from the waist opening towards the leg opening from above to below in the vertical direction and from inside to outside in the lateral direction,
when the underpants-shaped absorbent article in a stretched state is viewed along the front-back direction,
in a vertical lower end portion of the joining portion, the underpants-shaped absorbent article has a portion where at least one of the absorbent main body and the belt portion protrudes toward a side of the leg opening.

16. An underpants-shaped absorbent article having a vertical direction, a lateral direction, and a front-back direction that intersect with each other,
the underpants-shaped absorbent article comprising:
an absorbent main body; and
a belt portion that connects a front upper end portion and a back upper end portion of the absorbent main body,
the underpants-shaped absorbent article having a waist opening and a pair of leg openings,
the belt portion being joined to the absorbent main body by a joining portion that inclines from the waist opening towards the leg opening from above to below in the vertical direction and from inside to outside in the lateral direction,
the joining portion being arranged so that during putting on a gap is formed between a lower end portion of the joining portion and a wearer's skin by the lower end portion of the joining portion rising up from a wearer's skin side to a wearer's non-skin side.
